(19) Europäisches Patentamt
European Patent Office
Office européen des brevets

(11) Publication number: **0 330 469 A2**

(12) **EUROPEAN PATENT APPLICATION**

(21) Application number: **89301773.1**

(22) Date of filing: **23.02.89**

(51) Int. Cl.[4]: **C 07 D 217/14**
C 07 D 401/06,
C 07 D 409/06, A 61 K 31/47

(30) Priority: **23.02.88 GB 8804107**
**12.07.88 GB 8816550**

(43) Date of publication of application:
**30.08.89 Bulletin 89/35**

(84) Designated Contracting States:
**AT BE CH DE ES FR GB GR IT LI LU NL SE**

(71) Applicant: **GLAXO GROUP LIMITED**
**Clarges House 6-12 Clarges Street**
**London W1Y 8DH (GB)**

(72) Inventor: **Naylor, Alan**
**35 Layston Park**
**Royston Hertfordshire (GB)**

**Bradshaw, John**
**1 Putty Hall Cottages Barley**
**Royston Hertfordshire (GB)**

**Bays, David Edward**
**9 Windmill Field**
**Ware Hertfordshire (GB)**

**Hayes, Ann Gail**
**5 Sandringham Road**
**Potters Bar Hertfordshire (GB)**

**Judd,Duncan Bruce**
**St.Margarets Road 24**
**Stanstead Abbots Ware Hertfordshire (GB)**

(74) Representative: **Pett, Christopher Phineas et al**
**Frank B. Dehn & Co. European Patent Attorneys Imperial House 15-19 Kingsway**
**London WC2B 6UZ (GB)**

Claims for the following Contracting States: ES + GR.

(54) **Tetrahydroisoquinoline derivatives.**

(57) Disclosed are compounds of formula (I) :

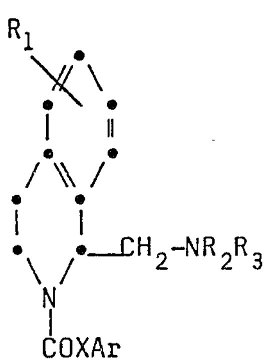

(I)

wherein
$R_1$ represents hydroxy or $C_{1-6}$ alkoxy;
$R_2$ and $R_3$ are the same or different and are $C_{1-6}$ alkyl or $C_{3-6}$ alkenyl or $-NR_2R_3$ represents a 5-membered (optionally containing an oxygen atom adjacent to the nitrogen) or a 6-membered ring, which ring optionally contains one unit of unsaturation and which is unsubstituted or substituted by hydroxy, oxo, optionally substituted methylidene, $-COR_4$(where $R_4$ is $C_{1-6}$ alkyl, aryl or ar($C_{1-6}$)alkyl) or $=NOR_5$ (where $R_5$ is $C_{1-6}$ alkyl);
X represents a direct bond, $-CH_2-$ or $-CH_2O$;
Ar represents a substituted phenyl moiety
and physiologically acceptable salts thereof.

Also described are methods for their preparation and pharmaceutical formulations thereof. The compounds are Kappa agonists.

EP 0 330 469 A2

## Description

### TETRAHYDROISOQUINOLINE DERIVATIES

This invention relates to tetrahydroisoquinoline derivatives, to processes for their preparation, to pharmaceutical compositions containing them and to their medical use. In particular, the invention relates to compounds which act as agonists at kappa opioid receptors.

Compounds which are kappa opioid receptor agonists have been indicated in the art for the treatment of a number of conditions and have been described, for example, as analgesics, as diuretics and in the treatment of cerebral ischaemia.

Opioid analgesia is generally thought to be mediated by either mu or kappa receptors in the brain (see, for example, Tyers M.B., Br. J. Pharmacol, (1980), 69, 503-512). Most existing clinically used opioid analgesics such as morphine and codeine act as mu-receptor agonists. However, these compounds have undesirable and potentially dangerous dependence forming side effects. There is thus a need for a strong analgesic with low dependence liability and a compound which is a selective kappa-receptor agonist would fulfil such a role.

Cerebral ischaemia or lack of blood flow in the brain, may result from a number of conditions, including, for example, stroke, head injuries or brain tumour. The resulting lack of oxygen to the brain cells causes neuronal damage and depending on the region of the brain involved, death or permanent disability may occur.

We have now found a novel group of tetrahydroisoquinoline derivatives which are selective kappa opioid receptor agonists. These compounds are therefore of interest in the treatment of conditions where the underlying aetiology indicates that treatment with a kappa opioid receptor agonist would be beneficial.

Thus, the present invention provides a compound of formula (I):

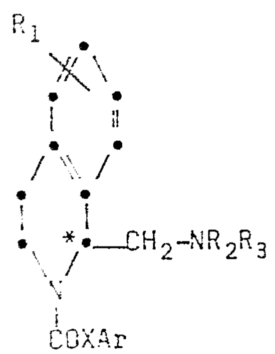

(I)

wherein

$R_1$ represents hydroxy or $C_{1-6}$ alkoxy;

$R_2$ and $R_3$ are the same or different and are $c_{1-6}$ alkyl or $C_{3-6}$ alkenyl or $-NR_2R_3$ represents a 5-membered (optionally containing an oxygen atom adjacent to the nitrogen) or a 6-membered ring, which ring optionally contains one unit of unsaturation and which is unsubstituted or substituted by hydroxy, oxo, optionally substituted methylidene, $-COR_4$(where $R_4$ is $C_{1-6}$ alkyl, aryl or ar($C_{1-6}$)alkyl) or $=NOR_5$ (where $R_5$ is $C_{1-6}$ alkyl);

X represents a direct bond, $-CH_2-$ or $-CH_2O$;

Ar represents a substituted phenyl moiety

and physiologically acceptable salts thereof.

As used herein, a $C_{1-6}$ alkyl group or the alkyl moiety of an ar($C_{1-6}$)alkyl or $C_{1-6}$ alkoxy group may be straight or branched chain and is conveniently $C_{1-4}$ alkyl for example methyl. An aryl group or the aryl moiety of an ar($C_{1-6}$)alkyl is preferably phenyl.

A $C_{1-6}$ alkoxy group is conveniently methoxy.

It will be appreciated that where $-NR_2R_3$ represents a ring containing a unit of unsaturation, this will not be attached to a carbon atom adjacent to the nitrogen atom.

An alkenyl group may be a straight or branched chain group. Where $R_2$ and/or $R_3$ in the compounds of formula (I) represents an alkenyl group, it will be appreciated that a double bond will not be attached to the carbon atom adjacent to the nitrogen.

The term 'optionally substituted methylidene' within the definition of the compounds of formula (I) is intended to cover methylidene substituted by any substituent conventional in the art. In the compounds of formula (I), the methylidene group may conveniently be substituted to form a conjugated system. Suitable substitutents which form a conjugated system with the methylidene double bond include, for example, nitrile, phenyl, carboxyl and amido substituents. Alternatively, the methylidene group may conveniently be substituted by, for example, $C_{1-6}$ alkyl, an ar($C_{1-6}$)alkyl group such as phenethyl, a $C_{1-6}$ hydroxyalkyl group such as hydroxymethyl, a $C_{1-6}$ carboxyalkyl group such as $-CH_2CH_2CO_2CH_3$ or a $C_{1-6}$ amidoalkyl group such

as $-CH_2CH_2CONH_2$.

The term 'a substituted phenyl moiety' is intended to cover a phenyl moiety substituted by one or more conventional substituents in the art, which substituents may form a second ring optionally containing one or more units of unsaturation. In the compounds of formula (I), Ar conveniently represents a phenyl moiety which is substituted by one or more $C_{1-6}$ alkyl groups or electron-withdrawing substituents, or in which two adjacent substituents form a carbocyclic or heterocyclic ring. Suitable electron-withdrawing substituents include, for example, halogen (for example, fluorine, chlorine or bromine), $-CF_3$ or $-NO_2$. Where two substituents on the phenyl ring form a second ring, Ar may suitably represent naphthyl, for example, 1-naphthyl or 2-naphthyl, or benzothiophene. Ar preferably represents phenyl, substituted at the meta and/or para positions on the phenyl ring, by one or more halogens, for example, chlorine, and is typically a 3,4-dichlorophenyl moiety.

The substituent $R_1$ is preferably attached at the 5-, 6- or 7-position of the tetrahydroisoquinoline nucleus. In a particularly preferred class of compounds of formula (I), the substituent $R_1$ is attached at the 5-position.

$R_1$ preferably represents a hydroxy or methoxy group. Particularly preferred compounds of formula (I) are those in which $R_1$ represents a hydroxy group, in particular those wherein $R_1$ is 5-hydroxy.

$-NR_2R_3$ may suitably represent a substituted or unsubstituted tetrahydropyridine, or pyrrolidine ring. $R_2$ and $R_3$ may both represent $C_{1-4}$ alkyl groups.

In a preferred class of compounds of formula (I), $-NR_2R_3$ represents a pyrrolidine ring which is substituted by -OH or =O. Where the ring $-NR_2R_3$ is substituted, the substituent is preferably attached to the carbon atom β to the nitrogen atom.

X preferably represents $-CH_2-$.

A preferred group of compounds falling within the scope of formula (I) is that in which $R_1$ represents hydroxy and $-NR_2R_3$ represents pyrrolidine ring substituted by -OH or =O; and physiologically acceptable salts thereof. In this group of compounds, X preferably represents $-CH_2-$ and Ar represents halosubstituted phenyl. Particularly preferred compounds are those in which Ar represents chlorosubstituted phenyl.

Preferred compounds of the invention include :

2-[(3,4-Dichlorophenyl)acetyl]-1,2,3,4-tetrahydro-1-[(3-hydroxy-1-pyrrolidinyl)methyl]-5-isoquinolinol;

2-[(3,4-Dichlorophenyl)acetyl]-1,2,3,4-tetrahydro-1-[(3-oxo-1-pyrrolidinyl)methyl]-5-isoquinolinol;

and physiologically acceptable salts thereof.

A particularly preferred compound is :

2-[(3,4-Dichlorophenyl)acetyl]-1,2,3,4-tetrahydro-1-(1-pyrrolidinylmethyl)-5-isoquinolinol and its physiologically acceptable salts.

Compounds of formula (I) contain at least one chiral centre (shown in formula (I) by *) and may exist in more than one stereoisomeric form. The invention includes within its scope all enantiomers, diastereomers and mixtures thereof. The invention also embraces all geometric isomers of compounds of formula (I). The preferred stereoisomeric form at the chiral centre common to all compounds of formula (I) is that represented by formula Ia.

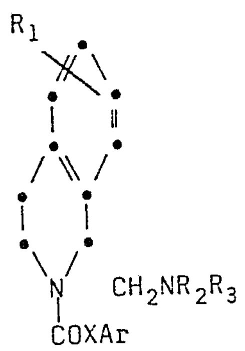

(Ia)

wherein $R_1$, $R_2$, $R_3$, X and Ar are as defined herein form formula (I).

Suitable physiologically aceptable salts are those conventionally known in the art. Examples of physiologically acceptable salts include acid addition salts formed with inorganic acids, such as hydrochlorides, hydrobromides, phosphates and sulphates, and with organic acids, for example tartrates, maleates, fumarates, succinates and sulphonates. Other salts which are not pharmaceutically acceptable may be useful in the preparation of compounds of formula (I) and these form a further part of the invention.

Compounds of formula (I) wherein $R_1$ represents a $C_{1-6}$alkoxy group are useful as intermediates in the preparation of compounds of formula (I) wherein $R_1$ represents a hydroxy group.

Compounds of the invention may readily be isolated in association with solvent molecules by crystallisation from or evaporation of an appropriate solvent. It is intended to include such solvates with the scope of the present invention.

Compounds falling within formula (I) have been shown to have analgesic activity using standard laboratory

animal tests such as the mouse acetylcholine writhing test (M. B. Tyers, Brit. J. Pharmacol, 1980, 69, 503-512) or the rat paw pressure test. Furthermore, their selective kappa receptor activity has been demonstrated in vitro in the field stimulated rabbit vas deferens preparation using the procedure described by A. G. Hayes and A Kelly, Eur. J. Pharmacol 110, 317-322 (1985). Compounds of the invention and their physiologically acceptable salts and solvates thus possess analgesic activity with the potential for low dependence liability and are therefore useful in the relief of pain.

Compounds of the invention are also of value in protecting against neuronal damage resulting from cerebral ischaemia which may be demonstrated for example in standard laboratory bilateral carotid occlusion models. Thus, compounds of the invention and their physiologically acceptable salts are also useful in treating or relieving the effects of cerebral ischaemia.

Accordingly, the invention also provides a compound of formula (I) or a physiologically acceptable salt thereof for use in medicine, in particular for the treatment of conditions where kappa agonists are indicated, (for example as analgesics and in the treatment of cerebral ischaemia).

In an alternative or further aspect there is provided a method of treatment of a mammal, including man, comprising administration of an effective amount of a compound of formula (I) or a physiologically acceptable salt thereof in particular in the treatment of conditions where the use of a kappa receptor agonist is indicated.

It will be appreciated that compounds of the invention will primarily be of use in the alleviation of established symptoms but prophylaxis is not excluded.

Compounds of the invention may be administered as the raw chemical but the active ingredient is preferably presented as a pharmaceutical formulation. The active ingredient may conveniently be presented in unit dose form.

According to another aspect, the invention provides a pharmaceutical composition comprising at least one compound of formula (I) or a physiologically acceptable salt or solvate thereof and formulated for administration by any convenient route conventional in the art. Such compositions are preferably in a form adapted for use in medicine, in particular human medicine and can conveniently be formulated in conventional manner using one or more pharmaceutically acceptable carriers or excipients. Compounds according to the invention may conveniently be formulated for oral or parenteral administration.

For oral administration, the pharmaceutical compositions may take the form of, for example, tablets or capsules prepared by conventional means with pharmaceutically acceptable excipients such as binding agents (for example pregelatinised maize starch, polyvinylpyrrolidone or hydroxypropyl methylcellulose); fillers (for example lactose, microcrystalline cellulose or calcium phosphate); lubricants (for example magnesium stearate, talc or silica); disintegrants (for example potato starch or sodium starch glycollate); or wetting agents (for example sodium lauryl sulphate). The tablets may be coated by methods well known in the art. Liquid preparations for oral administration may take the form of, for example, solutions, syrups or suspensions, or they may be presented as a dry product for constitution with water or other suitable vehicle before use. Such liquid preparations may be prepared by conventional means with pharmaceutically acceptable additives such as suspending agents (for example sorbitol syrup, methyl cellulose or hydrogenated edible fats); emulsifying agents (for example lecithin or acacia); non-aqueous vehicles (for example almond oil, oily esters or ethyl alcohol); and preservatives (for example methyl or propyl-p-hydroxybenzoates or sorbic acid).

The compounds of the invention may be formulated for parenteral administration by injection, preferably intravenous or subcutaneous injection, for example by bolus injection or continuous intravenous infusion. Where the compounds are administered by continuous intravenous infusion this is conveniently sequential to a bolus injection. Formulations for injection may be presented in unit dosage form, for example, in ampoules or in multi-dose containers, with an added preservative.

The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, for example sterile pyrogen-free water, before use.

It will be appreciated that the precise dose administered will depend on the age and condition of the patient, the particular compound used, and the frequency and route of administration. The compounds may be administered in single or divided doses and may be administered one or more times, for example 1 to 4 times, per day.

A proposed dose of the compounds of the invention for the relief of pain or the treatment of cerebral ischaemia is is 0.01 to 100 mg/kg body weight, preferably 0.01 to 10 mg/kg body weight, most preferably 0.1 to 10 mg/kg body weight per day.

The invention also provides the use of a compound of formula (I) or a physiologically acceptable salt thereof for the manufacture of a medicament for the treatment of conditions where kappa receptor agonists are indicated.

According to another aspect of the invention, compounds of formula (I) and physiologically acceptable salts and solvants, thereof may be prepared by the general methods outlined below. In the following methods, $R_1$, $R_2$, $R_3$, X and Ar are as defined for formula (I) unless otherwise indicated.

It will be appreciated that in the methods for preparing compounds of formula (I) given below, it may be necessary or desirable to protect one or more sensitive groups in the molecule to prevent undesirable side reactions. Thus, a reaction step involving deprotection of a protected derivative of a compound of the invention

may be required subsequent to any of the processes described below. Protection and deprotection may be effected using conventional procedures as described, for example, in 'Protective Groups in Organic Synthesis', T. W. Greene (John Wiley & Sons, 1981).

According to one general process (A), compounds of formula (I) may be prepared by reacting a compound of formula (II).

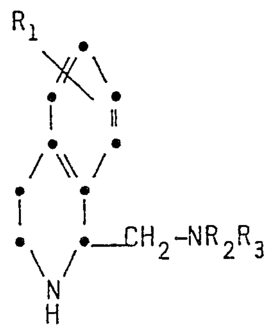

(II)

with a reagent serving to introduce the group -COXAr.

Thus, for example, compounds of formula (I) may be prepared by reacting a compound of formula (II) with an acid $ArXCO_2H$ or an acylating agent corresponding thereto or a salt thereof.

Suitable acylating agents corresponding to the acid $ArXCO_2H$ which may conveniently be used include, for example, acid halides (for example acid chlorides), alkyl esters (for example, methyl or ethyl esters) and mixed anhydrides. Such acylating agents may conveniently be prepared from the acid itself by conventional methods.

The reaction of a compound of formula (II) with an acid $ArXCO_2H$ is desirably effected in the presence of a coupling agent such as carbonyl diimidazole or diphenylphosphoryl azide in a suitable reaction medium and conveniently at a temperature of from -50 to +50°C, preferably at room temperature. The reaction may be effected in a suitable reaction medium such as an ether (for example tetrahydrofuran), a haloalkane (for example, dichloromethane), a nitrile (for example acetonitrile), an amide (for example dimethylformamide) or mixtures thereof.

The reaction of a compound of formula (II) with an acylating agent corresponding to the acid $ArXCO_2H$ may conveniently be effected using the reaction conditions described above and optionally in the presence of a base. Suitable bases which may be employed include, for example, organic bases such as pyridine or triethylamine or inorganic bases such as calcium carbonate or sodium bicarbonate.

Compounds of formula (II) may be prepared, for example, from compounds of formula (III):

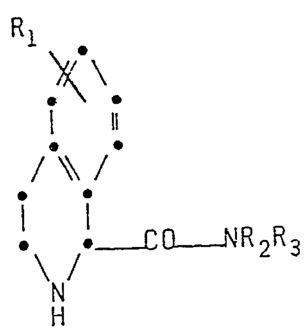

(III)

by reduction using a suitable reducing agent, for example, a metal hydride such as lithium aluminium hydride in a solvent such as tetrahydrofuran.

Compounds of formula (III) may themselves be prepared, for example, from the corresponding carboxylic acid (or an acylating agent corresponding thereto such as an alkyl ester, for example, the ethyl ester) by reaction with an amine $HNR_2R_3$ in a method analogous to that of process (A) above. The corresponding acid or ester may itself conveniently be prepared by reduction of the appropriate isoquinoline using, for example, hydrogen and a metal catalyst such as platinum oxide which may be supported, for example, on charcoal. The corresponding isoquinoline derivatives are either known compounds or may be prepared from known phenethylamine derivatives by conventional cyclisation procedures.

According to another general process (B), compounds of formula (I) may be prepared by reductive amination of a compound of formula (IV)

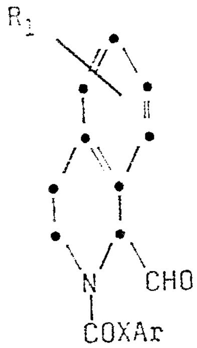

(IV)

with an amine $R_2R_3NH$ in the presence of a suitable reducing agent.

The reduction may be effected using an alkali metal or alkaline earth metal borohydride or cyanoborohydride (for example sodium borohydride or cyanoborohydride) in a suitable solvent, for example an alcohol such as methanol and at a suitable temperature, conveniently room temperature. The reaction may optionally be performed in the presence of an acid such as acetic acid.

Alternatively, the reduction may be effected catalytically, for example, using hydrogen in the presence of a metal catalyst such as Raney nickel, platinum, platinum oxide, palladium or rhodium which may be supported, for example, on charcoal. The reaction may conveniently be carried out in a suitable solvent such as an alcohol (for example ethanol), an amide (for example dimethylformamide) an ether (for example tetrahydrofuran) and at a suitable temperature.

Compounds of formula (IV) may be prepared, for example, from compounds of formula (V)

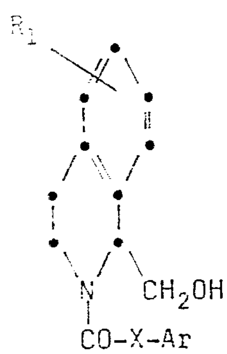

(V)

by oxidation using conventional methods, for example using an oxidising agent such as chromium trioxide in a suitable solvent, for example pyridine.

Compounds of formula (V) may themselves be prepared from the corresponding 1-unsubstituted piperidine of formula (VI)

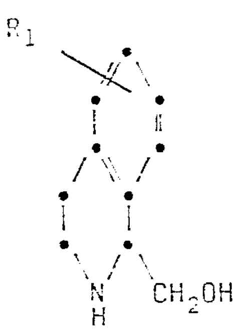

(VI)

by methods analogous to those described for general process (A) above. Compounds of formula (VI) are either known or can be prepared from known compounds by conventional methods.

According to a further general process (C), a compound of formula (I) according to the invention may be converted into another compound of the invention using conventional procedures.

According to one embodiment of process (C), a compound of formula (I) containing an oxo group may be converted into the corresponding optionally substituted methylidene derivative by reaction with an appropriate Wittig reagent, for example a phosphonate (such as trimethylphosphonoacetate) or a phosphorane prepared by reacting an appropriate triarylphosphonium salt (such as methyltripenylphosphonium bromide) with a base. Suitable bases which may be used include, for example, alkali metal hydrides such as sodium hydride, alkali metal alkoxides such as sodium or potassium t-butoxide or alkali lithiums such as n-butyl lithium. The reaction may conveniently be carried out in a solvent such as an ether, for example tetrahydrofuran, and at a temperature of from -70° to +50°.

Compounds of formula (I) in which $R_1$ represents an hydroxyl group may be prepared by cleavage of the corresponding alkoxy derivative using standard conditions. Thus, for example, the corresponding alkoxy may be treated with boron tribromide-dichloromethane in a solvent such as dichloromethane.

Compounds of formula (I) in which $-NR_2R_3$ represents a hydroxy-substituted ring can conveniently be prepared by reduction of the corresponding oxo compound using a suitable reducing agent such as an alkali metal borohydride or cyanoborohydride (for example sodium borohydride) or a metal hydride (for example diisobutyl aluminium hydride or lithium aluminium hydride) in a suitable solvent (for example, an alcohol such as ethanol or a hydrocarbon solvent such as toluene).

Compounds of formula (I) containing an oxo group may be prepared by oxidation of the corresponding alcohol using a suitable oxidising agent, for example an acid anhydride or acid chloride complex with dimethylsulphoxide (such as oxalylchloride- dimethylsulphoxide) in a solvent such as dichloromethane, conveniently at low temperature, followed by treatment with a base such as triethylamine.

Compounds of formula (I) containing an oxime substituent may conveniently be prepared from the corresponding oxo derivative by conventional oximation procedures, for example by reaction with an appropriate amine $R_5ONH_2$ in a suitable solvent such as pyridine, conveniently at room temperature.

An amido substituent may be prepared from the corresponding carboxy substituted compound of formula (I) according to the method of general process (A) above.

As well as being employed as the last main step in the reaction sequence, the general methods discussed above may also be used to introduce a desired group at any intermediate stage in the preparation of compounds of formula (I). Thus, for example, the required group at the 1-position of the tetrahydroisoquinoline ring in the compounds of the invention can be introduced before or after acylation to introduce the -COXAr moiety. It will be appreciated that the sequence of reactions will be chosen such that the reaction conditions do not affect groups present in the molecule which are required in the final product.

The general processes described above may yield the product of the general formula (I) as in individual stereoisomer or as a mixture of stereoisomers. Diastereoisomers may be separated at any convenient point in the overall synthesis by conventional methods e.g. chromatography. Specific enantiomers may be obtained by resolution of a racemic mixture at any convenient point in the overall synthesis by the use of conventional methods, see for example "Stereochemistry of Carbon Compounds" by E.L. Eliel (McGraw Hill, 1962).

Where it is desired to isolate a compound of the invention as a salt, thus may be formed by conventional methods, for example by treatment with an acid or base in a suitable solvent such as an ether (for example diethyl ether), a nitrile (for example acetonitrile), a ketone (for example acetone) a halogenated hydrocarbon (for example dichloromethane), or an ester (for example ethyl acetate). Salts may also be formed by conversion of one salt into another using conventional methods.

Solvates of compounds of formula (I) may conveniently be prepared by crystallisation or recrystallisation from an appropriate solvent.

The invention is further illustrated by the following examples.

All temperature are in °C. Chromatography was carried out in the conventional manner using silica gel (Merck, 7729) or by flash column chromatography on silica (Merck 9385) and thin layer chromatography (t.l.c) on silica except where otherwise stated. Dried refers to drying with $Na_2SO_4$ unless otherwise indicated.

Examples 1 and 2

1. 2-[(3,4-Dichlorophenyl)acetyl]-1,2,3,4-tetrahydro-7-methoxy-1-(1-pyrrolidinylmethyl)isoquinoline maleate (1:1)

2. 2-[(3,4-Dichlorophenyl)acetyl]1,2,3,4-tetrahydro-6-methoxy-1-(1-pyrrolidinylmethyl)isoquinoline maleate (1:1)

(i) Ethyl [[2-(4-methoxyphenyl)ethyl]amino]oxoacetate

A mixture of p-methoxyphenethylamine (3.78g) and diethyl oxalate (20.1g) was stirred at 120°C for 17.5h, the excess ester was removed in vacuo and the residue was triturated under ether (25mℓ). The resulting solid was filtered off and the filtrate evaporated to dryness to give an oil (6.16g). A portion of the oil (1g) was purified by flash column chromatography on silica gel eluting with ether-hexane (9:1), to give the title compound (830mg) as an oil.

| | | | | |
|---|---|---|---|---|
| Analysis | Found : | C,62.20; | H,6.80; | N,5.55. |
| $C_{13}H_{17}NO_4$ | requires | C,62.15; | H,6.80; | N,5.55%. |

Following the method of stage (i) m-methoxyphenethylamine (1.5g) was reacted with diethyl oxalate (8.05g) to give ethyl [[2-(3-methoxyphenyl)ethyl]amino]oxoacetate as an oil (1.76g).

| | | | | |
|---|---|---|---|---|
| Analysis | Found : | C,62.45; | H,6.85; | N,5.55. |
| $C_{13}H_{17}NO_4$ | requires | C,62.15; | H,6.80; | N,5.55% |

(ii) Ethyl 3,4-dihydro-7-methoxy-1-isoquinolinecarboxylate

Phosphorus oxychloride (4mℓ) was added to a solution of the product of stage (i) (1.76g) in toluene (17mℓ) and ethanol (1.4mℓ) and the mixture was heated at 120°C in an autoclave for 3.5h. After cooling to room temperature the solvent was evaporated and water (60mℓ) was added dropwise to the residue with cooling in an ice bath. Solid potassium carbonate was added until the mixture was approximately pH9.5. The mixture was extracted with ether (3 x 50mℓ) and the combined extracts were dried and evaporated to give an oil (1.93g). This oil was purified by column chromatography on silica gel eluting with ether-hexane (9:1) to give impure title compound (1.06g) a portion of which (53mg) was further purified by dissolving in ether (10mℓ) and extracting with dilute hydrochloric acid (0.2M, 3 x 5mℓ). The combined acid extracts were washed with ether (10mℓ) then basified with aqueous sodium carbonate (2N, 4mℓ) and extracted with ether (3 x 5mℓ). The ether extracts were combined, dried and evaporated to give the title compound (28mg) as an oil.

| | | | | |
|---|---|---|---|---|
| Analysis | Found: | C,66.55; | H,6.45; | N,6.25. |
| $C_{13}H_{15}NO_3$ | requires | C,66.95; | H,6.50; | N,6.00%. |

Ethyl 3,4-dihydro-6-methoxy-1-isoquinolinecarboxylate as an oil (801mg) was similarly prepared from ethyl [[2-(3-methoxyphenyl)ethyl]-amino]oxoacetate (1.6g) and phosphorus oxychloride (3.5mℓ).

| | | | | |
|---|---|---|---|---|
| Analysis | Found : | C,66.50; | H,6.25; | N,5.85. |
| $C_{13}H_{15}NO_3$ | requires | C,66.95; | H,6.50; | N,6.00% |

(iii) Ethyl 1,2,3,4-tetrahydro-7-methoxy-1-isoquinolinecarboxylate

A solution of the product of stage (ii) (3.6g) in glacial acetic acid (50mℓ) was hydrogenated over platinium oxide (200mg) at 60psi at room temperature for 18h. The catalyst was filtered off and the filtrate and washings were combined and evaporated in vacuo to give an oil. The oil was dissolved in water (50mℓ) and the solution was basified with aqueous sodium carbonate (2N, 130mℓ). The solution was extracted with chloroform (3 x 50mℓ) and the extracts were combined, dried and evaporated to give an oil (3.17g). Purification by flash column chromatography on silica gel eluting with ether-methanol (19:1) gave the title compound (2.21g) as an oil.

T.l.c. Silica/ether-methanol (19.1) Rf 0.23 detection u.v., IPA, alkaline $KMnO_4$).

Following the method of stage (iii), ethyl 3,4-dihydro-6-methoxy-1-isoquinolinecarboxylate (800mg) was hydrogenated to give ethyl 1,2,3,4-tetrahydro-6-methoxy-1-isoquinoline carboxylate as an oil (648mg).

| | | | | |
|---|---|---|---|---|
| Analysis | Found : | C,66.10; | H,7.40; | N,5.95. |
| $C_{13}H_{17}NO_3$ | requires : | C,66.35; | N,7.30; | N,5.95% |

(iv) 1-[(1,2,3,4-Tetrahydro-7-methoxy-1-isoquinolinyl)carbonyl] pyrrolidine

A solution of the product of stage (iii) (2.08g) in pyrrolidine (4.5mℓ) was heated at 150°C for 4h in an autoclave. Further pyrrolidine (4.5mℓ) was added and the mixture was re-heated at 150°C for 2h. After cooling to room temperature the mixture was evaporated in vacuo to give an oil (2.66g). This oil was purified by flash column chromatography on silica gel eluting with chloroform-methanol (19:1) to give impure title compound as an oil (1.73g). Purification by chromatography on silica gel eluting with dichloromethane-methanol (9:1) gave the title compound (1.44g) as an oil.

| | | | | |
|---|---|---|---|---|
| Analysis | Found: | C,65.25; | H,7.55; | N,10.00. |
| $C_{15}H_{20}N_2O_2.0.23CH_2Cl_2$ | requires | C,65.35; | H,7.35; | N,10.00%. |

Following the method of stage (iv), ethyl 1,2,3,4,-tetrahydro-6-methoxy-1-isoquinoline carboxylate (559mg) and pyrrolidine (total of 2.5mℓ) were reacted to give 1-[(1,2,3,4-tetrahydro-6-methoxy-1-isoquinolinyl)carbonyl]pyrrolidine as a solid (540mg), m.p. 100-103°.

(v) 1,2,3,4-Tetrahydro-7-methoxy-1-(1-pyrrolidinylmethyl) isoquinoline

A solution of the product of stage (iv) (1.38g) in dry tetrahydrofuran (15mℓ) was added dropwise to a stirred suspension of lithium aluminium hydride (600mg, 15.8mmol) in dry tetrahydrofuran (30mℓ) under nitrogen. After stirring for 30 min. the mixture was heated under reflux for 17h, cooled in an ice-water bath and water (22.5mℓ) was cautiously added dropwise. Aqueous sodium hydroxide (2N, 7.5mℓ) was added followed by chloroform (40mℓ) and the insoluble material was filtered off. The filtrate layers were separated and the chloroform layer was dried and evaporated to give the title compound (1.24g) as an oil.

| | | | | |
|---|---|---|---|---|
| Analysis | Found: | C,70.25; | H,9.00; | N,10.75. |
| $C_{15}H_{22}N_2O.0.1CHCl_3$ | requires | C,70,25; | H,8.60; | N,10.85%. |

1,2,3,4-Tetrahydro-6-methoxy-1-(1-pyrrolidinylmethyl)isoquinoline was similarly prepared as an oil (380mg) from 1-[(1,2,3,4-tetrahydro-6-methoxy-1-isoquinolinyl)carbonyl]pyrrolidine (420mg) and lithium aluminium hydride (184mg).

| | | | | |
|---|---|---|---|---|
| Analysis | Found : | C,70.70; | H,8.65; | N,11.15. |
| $C_{15}H_{22}N_2O$ | requires : | C,73.15; | H,9.00; | N,11.40% |

(vi) 2-[(3,4-Dichlorophenyl)acetyl]-1,2,3,4-tetrahydro-7-methoxy-1-(1-pyrrolidinyl methyl)isoquinoline maleate (1:1)

A solution of 1,1′-carbonyldiimidazole (940mg) in warm, dry acetonitrile (10mℓ) was added to a solution of 3,4-dichlorophenylacetic acid (1.08g) in dry acetonitrile (20mℓ) under dry nitrogen and the mixture was stirred at room temperature under nitrogen for 1.5h. A solution of the product of stage (v) (1.18g) in dry acetonitrile (8mℓ) was added and stirring under nitrogen was continued for 17h. The mixture was evaporated to dryness and the residue was partitioned between chloroform (150mℓ) and aqueous sodium carbonate (2N, 50mℓ). The chloroform layer was washed with aqueous sodium carbonate (2N, 50mℓ) and water (2x50mℓ), dried and evaporated to give an oil (2.7g). This oil was purified by flash column chromatography on silica gel eluting with dichloromethane-methanol (19:1) followed by dichloromethane-methanol (9:1) to give the free base of the title compound (1.62g) as a foam.

A portion of the foam (435mg) was dissolved in ethyl acetate (4.5mℓ) and added to a solution of maleic acid (128mg) in ethyl acetate (6.5mℓ) giving on trituration the title compound (459mg) as a solid m.p. 186-187°C

| | | | | |
|---|---|---|---|---|
| Analysis | Found: | C,58.95; | H,5.70; | N,4.85. |
| $C_{23}H_{26}Cl_2N_2O_2.C_4H_4O_4.0.25C_4H_8O_2$ | requires | C,58.85; | H,5.65; | N,4.90%. |

2-[(3,4-Dichlorophenyl)acetyl]-1,2,3,4-tetrahydro-6-methoxy-1-(1-pyrrolidinylmethyl)isoquinoline maleate (1:1) (Example 2) was similarly prepared as solid (487mg) from 1,2,3,4-tetrahydro-6-methoxy-1-(1-pyrrolidinylmethyl)isoquinoline (360mg). m.p. 173-175°C.

| | | | | |
|---|---|---|---|---|
| Analysis | Found : | C,58.90; | H,5.60; | N,5.00. |
| $C_{23}H_{26}C\ell_2N_2O_2.C_4H_4O_4$ | requires | C,59.00; | H,5.50; | N,5.10% |

Example 3

2-[(3,4-Dichlorophenyl)acetyl]-1,2,3,4-tetrahydro-1-(1-pyrrolidinyl methyl)-7-isoquinolinol hydrobromide

A mixture of the product of Example 1 (0.35g) in 8% sodium bicarbonate solution (25mℓ) was extracted with dichloromethane (3x25mℓ), dried ($MgSO_4$) and evaporated to give the free base as an oil (0.28g). A 1M solution of boron tribromide in dichloromethane (1.24mℓ) was added to an ice-cooled stirred solution of the free base (0.28g) in dry dichloromethane and the reaction mixture was allowed to warm up to room temperature and then allowed to stand for 18h. Methanol (25mℓ) was added to the ice-cooled solution which was heated under reflux for 2h and evaporated to dryness. The residue was triturated under dry ether to give a solid (250mg) which was crystallised from ethyl acetate/methanol to give the title compound as a solid (80mg) m.p. 245-8°.

T.l.c. Silica ethylacetate/methanol (9:1).

| | | | | |
|---|---|---|---|---|
| Analysis | Found: | C,50.9; | H,4.9; | N,5.1. |
| $C_{22}H_{24}Cl_2N_2O_2.HBr.H_2O$ | | C,51.0; | H,5.25; | N,5.4%. |

Example 4

2-[(3,4-Dichlorophenyl)acetyl]-1,2,3,4-tetrahydro-1-(1-pyrrolidinylmethyl)-6-isoquinolinol hydrobromide

A solution of the product of Example 2 (0.35g) in 8% sodium bicarbonate solution (25mℓ) was extracted with dichloromethane (3x25mℓ), dried ($MgSO_4$) and evaporated to dryness to give the free base (0.26g). A 1M solution of boron tribromide in dichloromethane (1.2mℓ, 0.0012mol) was added to a solution of the free base (0.26g) in dry dichloromethane (25mℓ) at -10°C and the solution was allowed to warm up to room temperature and was left for 18h. Methanol (20mℓ) was added to the ice-cooled solution which was heated under reflux for 1h. The solution was evaporated to dryness and the residue was triturated under dry ether to give a solid (250mg) which was crystallised from ethyl acetate/methanol to give the title compound as a solid (0.13g) m.p. 264-6°.

T.l.c. Silica, Ethylacetate/methanol (9:1) Rf 0.1.

| | | | | |
|---|---|---|---|---|
| Analysis | Found: | C,51.7; | H,4.9; | N,5.2. |
| $C_{22}H_{24}Cl_2N_2O_2.HBr.0.5H_2O$ | requires | C,51.9; | H,5.15; | N,5.5%. |

Example 5

2-[(4-Chlorophenoxy)acetyl]-1,2,3,4-tetrahydro-5-methoxy-1-(1-pyrrolidinylmethyl)isoquinoline

Following the method of Examples 1, stage (VI), 1,2,3,4-tetrahydro-5-methoxy-1-(1-pyrrolidinylmethyl)isoquinoline (0.29g) and 4-chlorophenoxyacetic acid (0.26g) gave the title compound as a foam after purification by flash column chromatography eluting with dichloromethane/ethanol/ammonia (200:8:1), m.p. 50° softens.

T.l.c. Silica Dichloromethane/methanol/ammonia 150/8/1 Rf 0.35.

Example 6

2-[(4-Chlorophenoxy)acetyl]-1,2,3,4-tetrahydro-1-(1-pyrrolidinylmethyl)-5-isoquinolinol

Following the method of Example 3 the product of Example 5 (0.385g) gave the title compound as a solid (0.09g) following purification by flash column chromatography eluting with dichloromethane/methanol/ammonia (200:8:1) and crystallisation from methylacetate and hexane, m.p. 94-98°.

| | | | | |
|---|---|---|---|---|
| Assay | found | C,64.31; | H,6.41; | N,6.72; |
| $C_{22}H_{25}ClN_2O_3$. 0.6 $H_2O$ | requires | C,64.05; | H,6.42; | N,6.79% |

Example 7

2-[(3,4-Dichlorophenyl)acetyl]-1,2,3,4-tetrahydro-1-[(3-hydroxy-1-pyrrolidinyl)methyl]-5-methoxyisoquinoline fumarate

(i) 1,2,3,4-Tetrahydro-5-methoxy-1-isoquinolinemethanol

A solution of ethyl 1,2,3,4-tetrahydro-5-methoxy-1-isoquinoline carboxylate (8.23g) in dry tetrahydrofuran (150mℓ) was added dropwise to a stirred suspension of lithium aluminium hydride (2.12g, 55.96mmol) in dry tetrahydrofuran (50mℓ) under nitrogen at 0°C. The resulting suspension was stirred at room temperature for 2.5h. The reaction mixture was quenched carefully by the addition of water (2mℓ), 5N sodium hydroxide solution (4mℓ), and water (2mℓ) and then filtered, washed with tetrahydrofuran and the filtrate evaporated to dryness. The residue was dissolved in dichloromethane, dried and evaporated to give a solid. The solid was crystallised from ethyl acetate/hexane to give the title compound as a solid (3.93g) m.p. 130-132°.

(ii) 2-[(3,4-Dichlorophenyl)acetyl]-1,2,3,4-tetrahydro-5-methoxy-1-isoquinolinemethanol

A solution of 3,4-dichlorophenylacetic acid (4.36g) in dry dichloromethane (100mℓ) was treated with 1,1'-Carbonyldiimidazole (3.28g) and the mixture was stirred at room temperature for 1h. The resulting solution was added dropwise to a suspension of the product of stage (i) (3.91g) in dry dichloromethane (60mℓ) and stirred at room temperature for 19h. The reaction mixture was diluted with dichloromethane (100mℓ) and washed with aqueous 2N sodium carbonate solution (2x200mℓ). The organic layer was dried and evaporated to give a solid which was crystallised from ethyl acetate/methanol/hexane to give the title compound as a solid (5.12g) m.p. 173-176°.

(iii) 2-[(3,4-Dichlorophenyl)acetyl]-1,2,3,4-tetrahydro-5-methoxy-1-isoquinolinecarboxaldehyde

A solution of dimethylsulphoxide (1.23g) in dry dichloromethane (10mℓ) was added to a stirred solution of oxalyl chloride (1.06g) in dry dichloromethane (25mℓ) at -56°C under nitrogen. The resulting solution was stirred at -55 - -60°C for 30min followed by dropwise addition of the product of stage (ii) (2.5g) in dry dichloromethane (30mℓ). The reaction mixture was stirred under nitrogen at -56°C for 2h. Triethylamine (3.26g, 4.49mℓ) was added and the mixture was allowed to warm to -20°C, then quenched with water (50mℓ). The layers were separated and the aqueous layer was further extracted with dichloromethane (2x60mℓ). The combined organic extracts were dried and evaporated to give an oil (3.02g).
T.l.c. ($SiO_2$) $CH_2Cl_2$:MeOH:0.880 $NH_3$ (100:8:1), Rf 0.64.

(iv) 2-[(3,4-Dichlorophenyl)acetyl]-1,2,3,4-tetrahydro-1-[(3-hydroxy-1-pyrrolidinyl)methyl]-5-methoxyisoquinoline fumarate

A suspension of the prouduct of stage (iii) (2.49g) in methanol (50mℓ) was added to a stirred suspension of 3-pyrrolidinol (689mg) and 3Å molecular sieves (3.0g) in methanol (15mℓ), which had been adjusted to pH 6 using methanolic hydrogen chloride solution. Sodium cyanoborohydride (850mg) was added portionwise and the resulting suspension was stirred under nitrogen for 22h. The suspension was filtered and the filtrate was evaporated to dryness. The residue was partioned between aqueous 2N sodium carbonate solution (100mℓ) and dichloromethane (100mℓ). The aqueous layer was further extracted with dichloromethane (2x60mℓ). The combined organic extracts were dried and evaporated to give an oil, which was purified by flash column chromatography eluting with dichloromethane:methanol:0.880 $NH_3$ (200:8:1) to give the free base of the title compound as a foam (583mg). A portion of the free base (50mg) in ethyl acetate was treated with a solution of fumaric acid (14mg) in a mixture of ethyl acetate and methanol. The resulting solid was washed with dry diethyl ether and crystallised from ethyl acetate/methanol to give the title compound as a solid (34mg) m.p. 196-200°.
T.l.c. ($SiO_2$) $CH_2Cl_2$:MeOH:0.880 $NH_3$ (100:8:1). Rf 0.36.

Example 8

2-[(3,4-Dichlorophenyl)acetyl]-1,2,3,4-tetrahydro-1-[(3-hydroxy-1-pyrrolidinyl)methyl]-5-isoquinolinol fumarate

Boron tribromide (1M solution in dichloromethane, 1.4mℓ, 1.4mmol) was added dropwise to a stirred solution of Example 7 (200mg) in dry dichloromethane (4mℓ) at -10° under nitrogen. The resulting solution was allowed to warm to room temperature and stirring was continued for 17h. The reaction mixture was cooled to -10°, quenched with methanol (6mℓ) and heated at reflux for 1h. Ammonium chloride (100mg) was added and the solvent was removed in vacuo. The residue was purified by flash column chromatography eluting with dichloromethane:methanol: 0.880$NH_3$ (80:8:1) to give the free base of the title compound as a form (138mg).

A portion of the free base (41mg) in ethyl acetate was treated with a solution of fumaric acid (12mg) in ethyl acetate/methanol. The resulting solid was triturated under dry diethyl ether, followed by hot methanol. The solid was filtered off and dried to give the title compound as a solid (25mg) decomp. 230°.

| | | | | |
|---|---|---|---|---|
| Analysis | Found: | C,57.50; | H,5.27; | N,5.43. |
| $C_{22}H_{24}Cl_2N_2O_3.0.8C_4H_4O_4$ | requires | C,57.30; | H,5.19; | N,5.30%. |

Example 9

2-[(3,4-Dichlorophenyl)acetyl]-,1,2,3,4-tetrahydro-5-methoxy-1-[(3-oxo -1-pyrrolidinyl)methyl]isoquinoline fumarate

A solution of dimethylsulphoxide (89mg) in dry dichloromethane (1mℓ) was added to a stirred solution of oxalyl chloride (72mg) in dry dichloromethane (3mℓ) at -55°C under nitrogen. The resulting solution was stirred at -55°C for 30min, followed by dropwise addition of Example 7 (170mg) in dry dichloromethane (2mℓ). The reaction mixture was stirred under nitrogen at -55°C for 2h. A further amount of the complex formed from dimethylsulphoxide (89mg) and oxalyl chloride (72mg) was added and stirring at -55°C was continued for 1h. Triethylamine (153mg) was added and the mixture was allowed to warm to -20°C, and quenched with water (5mℓ). The layers were separated and the aqueous layer was further extracted with dichloromethane (2x10mℓ). The combined organic extracts were dried and evaporated to give an oil, which was purified by flash column chromatography eluting with ethyl acetate to give the title compound as a colourless oil (98mg).

A solution of the free base (89mg) in ethyl acetate was treated with a solution of fumaric acid (25mg) in ethyl acetate/methanol. The resulting solid was triturated twice under dry diethyl ether and crystallised from ethyl acetate/hexane to give the title compound as a solid (24mg) m.p. 164-166°.
T.l.c. ($SiO_2$ ($CH_2Cl_2$:MeOH:0.880 $NH_3$ (100:8:1). Rf 0.63.

Example 10

2-(Benzo[b]thien-4-ylacetyl)-1,2,3,4-tetrahydro-5-methoxy-1-(1-pyrrolidinylmethyl)isoquinoline

A solution of 5-benzo(b)thiophenacetic acid (0.1g) and 1,1′-carbonyldimidazole (0.084g) in dry dichloromethane (3mℓ) was stirred at ambient temperature for 1h. A solution of 1,2,3,4-tetrahydro-5-methoxy-1-(1-pyrrolidinylmethyl)isoquinoline (0.12g) in dry dichloromethane (2mℓ) was added and the mixture was stirred at ambient temperature for 20h. The reaction mixture was diluted with dichloromethane (20mℓ), washed with aqueous sodium carbonate (2M; 15mℓ), dried filtered and evaporated to give a residue (0.23g). The residue was purified by flash column chromatography eluting with dichloromethane/methanol/ammonia 200:8:1 to give a solid (0.572g) a portion of which was crystallised from methyl acetate/hexane to give the title compound as a solid (36mg) m.p.144°.

| | | | | |
|---|---|---|---|---|
| Analysis | Found: | C,71.04; | H,6.74; | N,6.35. |
| $C_{25}H_{28}N_2O_2S$ | requires | C,71.40; | H,6.71; | N,6.60%. |

Example 11

2-(Benzo[b]thien-4-yl-acetyl)-1,2,3,4-tetrahydro-1-(1-pyrrolidinylmethyl)-5-isoquinolinol

A solution of the product of Example 10 (0.135g) in dry dichloromethane (15mℓ) at -10° was treated with a solution of boron tribromide in dichloromethane (1mℓ) and the mixture was stirred at -10° to ambient temperature for 5h. Methanol (10mℓ) was cautiously added and the mixture was heated at reflux for 1h. The solvent was removed in vacuo further methanol (10mℓ) was added and the mixture was heated at reflux for 4h. The solvent was removed in vacuo and the residue dissolved in dichloromethane (10mℓ) and washed with aqueous sodium bicarbonate solution (10mℓ). The aqueous solution was extracted with dichloromethane (3x10mℓ). The organic solution was dried and evaporated leaving an oily residue (70mg). The residue was purified by flash column chromatography eluting with dichloromethane:methanol:ammonia 200:8:1 to give the title compound as a foam (0.03g).

T.l.c. ($SiO_2$; Dichloromethane:methanol:ammonia 150:8:1 Rf 0.35.

Example 12

2-[(3.4-Dichlorophenyl)acetyl]-1,2,3,4-tetrahydro-1-(1-pyrrolidinylmethyl-5-isoquinolinol fumarate salt (2:1)

(i) 2-[(3.4-Dichlorophenyl)acetyl]-1,2,3,4-tetrahydro-5-methoxy-1-[(1,2,3,6-tetrahydro-1-pyridinyl)methyl]isoquinoline

A solution of the compound of Example 7, stage (iii) (1.2g) and tetrahydropyridine (0.38g; 4.5mmol) in dry methanol (15mℓ) was stirred at ambient temperature. The pH of the solution was adjusted to 6.5 with methanolic hydrogen chloride, and 3Å molecular sieves (0.5g) were added. The mixture was stirred for 30min sodium cyanoborohydride (0.42g) was added and the mixture was stirred for 18h at ambient temperature. The reaction mixture was filtered and the filtrate was evaporated in vacuo. The residue was dissolved in ethyl acetate (50mℓ) and washed with aqueous sodium carbonate (0.5M; 50mℓ), hydrochloric acid (1M; 50mℓ) and aqueous sodium carbonate (0.5M; 25mℓ). The organic solution was dried and evaporated in vacuo. The residue (1.2g), was purified by flash column chromatography eluting with dichloromethane/methanol/ammonia/300:8:1, followed by chromatography on alumina (Merck 1077 Grade 2) using ethyl acetate/hexane 1/1 as eluent, to give the title compound as a foam (0.14g).

T.l.c. $SiO_2$ dichloromethane/methanol/ammonia 150:8:1 Rf 0.4.

(ii) 2-[(3,4-Dichlorophenyl)acetyl]-1,2,3,4-tetrahydro-1-pyridinyl)methyl] -5-isoquinolinol fumarate salt (2:1)

A solution of the product of stage (i) (0.14g) in dry dichloromethane (25mℓ) at -15° was treated with a solution of borontribromide in dichloromethane (1M; 1.2mℓ; 1.2mmol). The mixture was stirred at -15° for 20min and then at ambient temperature for 3h. The reaction mixture was cooled to -40° and treated with methanol (20mℓ), the solvent was removed in vacuo and the residue was dissolved in methanol (20mℓ) and re-evaporated. The residue was dissolved in methanol (20mℓ) and treated with aqueous ammonia (1mℓ). The solvent was removed in vacuo and the residue was purified by flash column chromatography eluting with dichloromethane/methanol/ammonia 200:8:1 to give the free base of the title compound as a foam (0.11g). A solution of the free base in ethyl acetate (5mℓ) was treated with a solution of fumaric acid (25mg; 0.21mmol) in a mixture of methanol and ethyl acetate to give a solid which was crystallized from methanol to give the title compound as a solid (20mg) m.p. 224-7° dec.

T.l.c. $SiO_2$ dichloromethane/methanol/ammonia 150:8:1 Rf 0.6.

Example 14

(i) 2-[(3,4-Dichlorophenyl)acetyl]-1-[(dimethylamino)methyl]-1,2,3,4-tetrahydro-5-methoxyisoquinoline

A solution of the compound of Example 7 stage (iii) (1.2g) and dimethylamine hydrochloride (0.38g) in dry methanol (15mℓ) was stirred at ambient temperature. The pH of the solution was adjusted to 6.5 with methanolic hydrogen chloride, and 3Å molecular sieves (0.5g) were added. The mixture was stirred for 30min sodium cyanoborohydride (0.42g; 6.6mmol) was added and the mixture was stirred for 18h at ambient temperature. The reaction mixture was filtered and the filtrate was evaporated in vacuo. The residue was dissolved in ethyl acetate (50mℓ) and was washed with aqueous sodium carbonate (0.5M; 25mℓ), hydrochloric acid (1M; 50mℓ) and aqueous sodium carbonate (0.5M; 25mℓ). The organic solution was dried and evaporated in vacuo. The residue was purified by flash column chromatography eluting with dichloromethane/methanol/ammonia 450:8:1 to give the title compound as a gum (0.58g).
T.l.c. ($SiO_2$ dichloromethane/methanol/ammonia 150:8:1) Rf 0.25.

(ii) 2-[(3,4-Dichlorophenyl)acetyl]-1-[(dimethylamino)methyl]-1,2,3,4-tetrahydro-5-isoquinolinol fumarate (salt 2:1)

A solution of the product of stage (i) (0.42g) in dry dichloromethane (50mℓ) at -15° was treated with a solution of boron tribromide in dichloromethane (1M; 3mℓ, 3mmol), over a 5 minute period. The mixture was stirred for 10 minutes at -15° and then at ambient temperature for 3 hours. The reaction mixture was cooled to -40° and treated with methanol (10mℓ). The solvent was evaporated in vacuo and the residue was dissolved in methanol and re-evaporated. The residue was purified by flash column chromatography eluting with dichloromethane/methanol/ammonia 150:8:1) to give the free base of the title compound as a gum (0.072g). The gum was dissolved in ethyl acetate (2mℓ) and treated with a solution of fumaric acid (20mg; 0.17mmol) in a mixture of methanol and ethyl acetate. The solid was crystallised from ethyl acetate and methanol to give the title compound as a solid (0.075g), m.p. 204-8° dec.
T.l.c. $SiO_2$ $CH_2Cl_2$/MeOH/$NH_3$, 150:8:1, Rf 0.15

| | | | | |
|---|---|---|---|---|
| Assay A62063 | | C, 57.66; | H,5.66; | N,5.33; |
| $C_{20}H_{22}Cl_2N_2O_2$. 0.55 $C_4H_4O_4$. 0.57 $C_4H_8O_2$ | requires | C,57.95; | H,5.71; | N,5.52% |

Example 14

2-[(3,4-Dichlorophenyl)acetyl]-1,2,3,4-tetrahydro-1-[(3-oxo-1-pyrrolidinyl)methyl]-5-isoquinolinol

A solution of dimethylsulphoxide (198mg) in dry dichloromethane (3mℓ) was added to a stirred solution of oxalyl chloride (150mg) in dry dichloromethane (4mℓ) at -55°C under nitrogen. The resulting solution was stirred at -55°C for 20min, followed by dropwise addition of the compound of Example 8 (368mg) in dry dichloromethane (2mℓ). The reaction mixture was stirred at -60 - -55°C for 2.5h. Triethylamine (299mg) was added and the mixture was allowed to warm to -20°C, then quenched with water (0.5mℓ) and poured into a phosphate buffer (pH 6.5) (5mℓ). The aqueous layer was further extracted with dichloromethane (2x5mℓ) and the combined organic extracts were dried and evaporated to give an oil. This oil was purified by flash column chromatography eluting with dichloromethane:methanol:0.880 ammonia (200:8:1) to give the free base as a foam (157mg).

The free base (~66mg) was re-purified by flash column chromatography eluting with chloroform/propanol-2-ol (98:2) to give the title compound as a foam (28mg) softens 62°.
T.l.c. ($SiO_2$) deactivated $Et_3N$ 3% Propan-2-ol/chloroform Rf 0.35.
N.m.r. δ (DMSO-$D_6$) (413K) includes 2.23-2.34 (2H, t), 2.55-3.13 (8H, + $H_2O$, m) 3.38-3.55 (1H, m), 3.73-3.99 (2H, AB), 4.07-4.26 (1H, m), 5.39-5.54 (1H, m), 8.62-8.78 (1H brs).

Example 15

2-[(3,4-Dichlorophenyl)acetyl]-1,2,3,4-tetrahydro-5-methoxy-(1-pyrrolidinylmethyl)-5-isoquinolinol maleate

(i) Ethyl [2-(2-methoxyphenyl)ethyl]amino]oxoacetate

A mixture of 2-methoxyphenethylamine (18.36g) and diethyl oxalate (97.57g) was heated at 125°C for 21h under nitrogen. The excess diethyl oxalate was evaporated off and the residue was triturated under diethyl ether. The solid obtained was filtered off and the filtrate was evaporated to dryness to give an oil which was purified by flash chromatography on silica gel (Merck 7747) eluting with diethyl ether:hexane (9:1) to give the title compound as an oil (30.69g).
T.l.c. ($SiO_2$) ether:hexane (9:1) Rf 0.33.

(ii) Ethyl 3,4-dihydro-5-methoxy-1-isoquinolinecarboxylate

Phosphorus oxychloride (4.6mℓ) was added to a solution of the product of stage (i) (2.0g) in toluene (19mℓ) and ethanol (16mℓ) and the mixture was heated at 120°C in an autoclave for 5h. Further amounts of

phosphorus oxychloride (2.3mℓ) and ethanol (0.8mℓ) were added and the resulting mixture was heated for a further 3.5h. The solvent was removed in vacuo and water (70mℓ) was added carefully to the ice-cooled residue, followed by the careful addition of potassium carbonate until the mixture was at pH8. The aqueous layer was extracted with diethyl ether (3x100mℓ) and the combined ether extracts z were extracted with 0.2N hydrochloric acid (4x50mℓ). The combined aqueous extracts were washed with ether (70mℓ) and basified with 2N sodium carbonate solution to pH 10. The aqueous layer was extracted with diethyl ether (3x60mℓ) and the combined organic extracts were dried ($MgSO_4$) and evaporated to give an oil which was purified by flash column chromatography on silica gel eluting with ether:hexane (4:1) to give the title compound as an oil (186mg).
T.l.c. ($SiO_2$) ether Rf 0.47.

(iii) Ethyl 1,2,3,4-tetrahydro-5-methoxy-1-isoquinolinecarboxylate

The product of stage (ii) (350mg) in acetic acid (5mℓ) was hydrogenated over platinum oxide (80mg) at atmospheric pressure. The catalyst was filtered off and the filtrate evaporated to dryness. A solution of the residue in 1N hydrochloric acid (10mℓ) was washed with diethyl ether (2x10mℓ), the aqueous layer was basified with 2N sodium carbonate solution and extracted with dichloromethane (3x10mℓ). The combined organic extracts were dried and evaporated to give an oil which was purified by flash column chromatography on silica gel eluting with diethyl ether:methanol (97:3) to give the title compound as an oil (114mg) T.l.c. ($SiO_2$) ether:methanol (19:1) Rf 0.32.

(iv) 1-[(1,2,3,4-Tetrahydro-5-methoxy-1-isoquinolinyl)carbonyl] pyrrolidine

The product of stage (iii) (710mg) and pyrrolidine (1.50mℓ) were heated in an autoclave at 120° C for 9h. The pyrrolidine was removed in vacuo and the residue was purified by flash column chromatography on silica gel eluting with ether:methanol (9:1 to 8:2) to give the title compound as a solid (453mg) T.l.c. ($SiO_2$) ether:methanol (9:1) Rf 0.26.

(v) 1,2,3,4-Tetrahydro-5-methoxy-1-(1-pyrrolidinylmethyl) isoquinoline

A solution of the product of stage (iv) (425mg) in dry tetrahydrofuran (10mℓ) was added dropwise to a stirred suspension of lithium aluminium hydride (186mg) in dry tetrahydrofuran (5mℓ) under nitrogen and the resulting suspension was heated at reflux for 2h. The reaction mixture was cooled and carefully quenched with water (0.2mℓ), 5N sodium hydroxide (0.4mℓ) and water (0.2mℓ). The mixture was filtered and the filtrate was evaporated to dryness. The residue was dissolved in dichloromethane, dried and evaporated to give the title compound as an oil (381mg).
T.l.c. ($SiO_2$) dichloromethane:methanol (19:1) Rf 0-0.18.

(vi) 2-[(3,4-Dichlorophenyl)acetyl]-1,2,3,4-tetrahydro-5-methoxy-1-(1-pyrrolidinylmethyl)isoquinoline maleate

1.1′-Carbonyldiimidazole (322mg) was added to a stirred solution of 3,4-dichlorophenyl acetic acid (436mg) in dry dichloromethane (10mℓ) and the resulting solution was stirred at room temperature under nitrogen for 1h. The product of stage (v) (349mg) in dry dichloromethane (5mℓ) was added and stirring was continued for 18h. The reaction mixture was diluted with dichloromethane (20mℓ) and washed with 2N sodium carbonate (20mℓ x 3). The organic layer was dried and evaporated to give an oil which was purified by flash column chromatography on silica gel eluting with dichloromethane:methanol:0.880 ammonia (250:8:1) to give the free base of the title compound as a foam (566mg). A portion of the free base (100mg) in ethyl acetate was treated with a solution of maleic acid (29mg) in ethyl acetate to give a solid which was crystallised from ethyl acetate/methanol to give the title compound as a solid (75mg) m.p. 146-148°.

Example 16

2-[(3,4-Dichlorophenyl)acetyl]-1,2,3,4-tetrahydro-1-(1-pyrrolidinylmethyl)-5-isoquinolinol maleate

Boron tribromide (1M solution in dichloromethane, 1.28mℓ) was added dropwise to a stirred solution of the free base of the title compound of Example 15 (221mg) in dry dichloromethane (5mℓ) at -10° C under nitrogen. The resulting solution was allowed to warm to room temperature and stirring was continued for 3.5h. The reaction mixture was cooled to -10° C and quenched with methanol (5mℓ). Ammonium chloride (100mg) was added and the solvent was removed in vacuo. The residue was purified by flash column chromatography on silica gel (M & B, Sorbsil C60) eluting with dichloromethane: methanol:0.880 ammonia (100:8:1) to give the free base of the title compound as a foam (160mg). A sample of the free base (141mg) in ethyl acetate (1mℓ) was treated with a solution of maleic acid (43mg) in ethyl acetate (2mℓ) and the resulting oil was triturated under dry diethyl ether (3x5mℓ). The solvent was decanted off and the solid was crystallised from a mixture of ethyl acetate:methanol to give the title compound as a solid (96mg) m.p. 135-137°.
T.l.c. ($SiO^2$) dichloromethane:methanol:ammonia (100:8:1) Rf 0.23

The following examples illustrate pharmaceutical formulations containing 2-[(3,4-dichlorophenyl)acetyl]-1,2,3,4-tetrahydro-1-[(3-1-pyrrolidinylmethyl]-5-isoquinolinol as active ingredient. Other compounds of formula (1) may be formulated in a similar manner.

TABLETS FOR ORAL ADMINISTRATION

DIRECT COMPRESSION

| | mg/tablet |
|---|---|
| Active ingredient | 20 |
| Calcium Hydrogen Phosphate B.P. * | 75.5 |
| Croscarmellose sodium USP | 4 |
| Magnesium Stearate, B.P. | 0.5 |
| Compression weight | 100mg |

* of a grade suitable for direct compression

The active ingredient is sieved before use. The calcium hydrogen phosphate, croscarmellose sodium and active ingredient are weighed into a clean polythene bag. The powders are mixed by vigorous shaking then the magnesium stearate is weighed and added to the mix which is blended further. The mix is then compressed using a Manesty F3 tablet machine fitted with 5.5mm flat bevelled edge punches, into tablets with target compression weight of 100mg.

Tablets may also be prepared by other conventional methods such as wet granulation.

Tablets of other strengths may be prepared by altering the ratio of active ingredient to lactose or the compression weight and using punches to suit.

The tablets may be film coated with suitable film forming materials, such as hydroxypropyl methylcellulose, using standard techniques. Alternatively the tablets may be sugar coated.

INJECTION FOR INTRAVENOUS ADMINISTRATION

| | mg/ml |
|---|---|
| Active ingredient | 5 |
| Sodium Chloride BP | as required |
| Water for Injection BP 0.5 to 2mℓ | |

INTRAVENOUS INFUSION

| | |
|---|---|
| Dextrose 5% aqueous solution BP | 10-100ml |
| Active ingredient | 700mg |
| Sodium Chloride BP | as required |

For infusion at a rate of 700mg per hour.

Sodium chloride may be added to adjust the tonicity of the solution and the pH may be adjusted, using acid or alkali, to that of optimum stability and/or to facilitate solution of the active ingredient. Alternatively suitable buffer salts may be used.

The solution is prepared, clarified and filled into appropriate size ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen or other suitable gas.

**Claims**

1. A compound of formula (I)

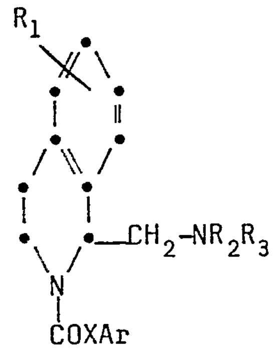

(I)

wherein
$R_1$ represents hydroxy or $C_{1-6}$ alkoxy;
$R_2$ and $R_3$ are the same or different and are $C_{1-6}$ alkyl or $C_{3-6}$ alkenyl or $-NR_2R_3$ represents a 5-membered (optionally containing an oxygen atom adjacent to the nitrogen) or a 6-membered ring, which ring optionally contains one unit of unsaturation and which is unsubstituted or substituted by hydroxy, oxo, optionally substituted methylidene, $-COR_4$(where $R_4$ is $C_{1-6}$ alkyl, aryl or ar($C_{1-6}$)alkyl) or $=NOR_5$ (where $R_5$ is $C_{1-6}$ alkyl);
X represents a direct bond, $-CH_2-$ or $-CH_2O$;
Ar represents a substituted phenyl moiety
and physiologically acceptable salts thereof.

2. A compound as claimed in Claim 1 wherein $R_1$ is hydroxyl.

3. A compound as claimed in Claim 1 or Claim 2 wherein $R_1$ is at the 6, or 7 position of the tetrahydroisoquinoline nucleus.

4. A compound as claimed in Claim 1 or Claim 2 wherein the substituent $R_1$ is at the 5-position of the tetrahydroisoquinoline nucleus.

5. A compound as claimed in any one of Claims 1 to 4 wherein $-NR_2R_3$ represents an unsubstituted or substituted pyrrolidine or tetrahydropyridine ring.

6. A compound according to any one of Claims 1 to 4 wherein $-NR_2R_3$ represents an unsubstituted pyrrolidine ring.

7. A compound according to any one of Claims 1 to 6 wherein X is $-CH_2-$.

8. A compound according to any one of Claims 1 to 7 wherein Ar is phenyl substituted at the 3, 4 or 3,4 positions by halogen.

9. A compound selected from :-
2-[(3,4-Dichlorophenyl)acetyl]-1,2,3,4-tetrahydro-1-[(3-hydroxy-1-pyrrolidinyl)methyl]-5-isoquinolinol;
2-[(3,4-Dichlorophenyl)acetyl]-1,2,3,4-tetrahydro-1-[(3-oxo-1-pyrrolidinyl)methyl]-5-isoquinolinol;
2-[(3,4-Dichlorophenyl)acetyl]-1,2,3,4-tetrahydro-5-methoxy-1-[(1-pyrrolidinyl)methyl]-5-isoquinolinol;
and physiologically acceptable salts thereof.

10. 2-[(3,4-Dichlorophenyl)acetyl]-1,2,3,4-tetrahydro-1-(1-pyrrolidinyl)methyl]-5-isoquinolinol and physiologically acceptable salts thereof.

11. A compound according to any of Claims 1 to 10 for use in therapy.

12. The use of a compound according to any of Claims 1 to 10 in the manufacture of a medicament for use in the treatment of pain or cerebral ischaemia.

13. A pharmaceutical composition which comprises a compound of formula (I) as defined in Claim 1 or a physiologically acceptable salt thereof together with a pharmaceutically acceptable carrier therefor.

**Claims for the following Contracting States: ES, GR**

1. A method for the preparation of a compound of formula (I) :

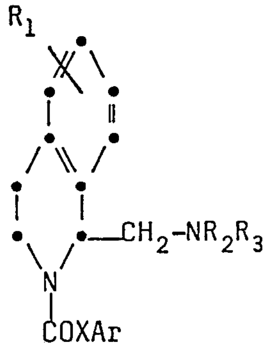

(I)

wherein
$R_1$ represents hydroxy or $C_{1-6}$ alkoxy;
$R_2$ and $R_3$ are the same or different and are $C_{1-6}$ alkyl or $C_{3-6}$ alkenyl or $-NR_2R_3$ represents a 5-membered (optionally containing an oxygen atom adjacent to the nitrogen) or a 6-membered ring, which ring optionally contains one unit of unsaturation and which is unsubstituted or substituted by hydroxy, oxo, optionally substituted methylidene, $-COR_4$(where $R_4$ is $C_{1-6}$ alkyl, aryl or ar($C_{1-6}$)alkyl) or $=NOR_5$ (where $R_5$ is $C_{1-6}$ alkyl);
X represents a direct bond, $-CH_2-$ or $-CH_2O$;
Ar represents a substituted phenyl moiety
and physiologically acceptable salts thereof.
which comprises :
(A) reacting a compound of formula (II)

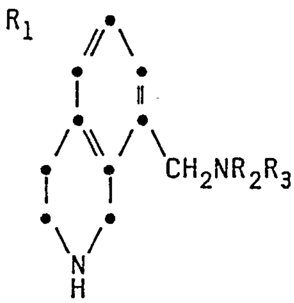

(II)

with a reagent serving to introduce the group -COXAr;
(B) reductive amination of a compound of formula (IV)

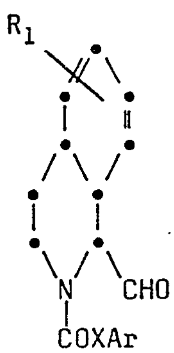

with an amine $R_2R_3NH$;
or
(C) subjecting another compound of formula (I) to an interconversion reaction;

and if necessary or desired subjecting the compound from any of the steps (A) to (C) to one or two further reactions comprising :

(i) converting a compound of formula (I) or a salt thereof into a physiologically acceptable salt thereof;

(ii) resolution of a racemic mixture to give a specific enantiomer.

2. A method as claimed in Claim 1 wherein $R_1$ is hydroxyl.

3. A method as claimed in Claim 1 or Claim 2 wherein $R_1$at in the 6 or 7 position of the tetrahydroisoquinoline nucleus.

4. A method as claimed in Claim 1 or Claim 2 wherein $R_1$ at in the 5 position of the tetrahydroisoquinoline nucleus.

5. A method as claimed in any one of Claims 1 to 4 wherein $-NR_2R_3$ represents an unsubstituted or substituted pyrrolidine or tetrahydropyridine ring.

6. A method as claimed in any one of Claims 1 to 4 wherein $-NR_2R_3$ is an unsubstituted pyrrolidine ring.

7. A method according to any one of Claims 1 to 6 wherein X is $-CH_2-$.

8. A method according to any one of Claims 1 to 7 wherein Ar is phenyl substituted at the 3, 4 or 3,4 positions by halogen.

9. A method as claimed in Claim 1 wherein the compound of formula (I) is :
2-[(3,4-Dichlorophenyl)acetyl]-1,2,3,4-tetrahydro-1-[(3-hydroxy-1-pyrrolidinyl)methyl]-5-isoquinolinol;
2-[(3,4-Dichlorophenyl)acetyl]-1,2,3,4-tetrahydro-5-methoxy-1-pyrrolidinyl)methyl]-5-isoquinolinol;
2-[3,4-Dichlorophenyl)acetyl]-1,2,3,4-tetrahydro-5-methoxy-1-[(1-pyrrolidinyl)methtyl]-5-isoquinolinol;
or a physiologically acceptable salt thereof.

10. A method as claimed in Claim 1 wherein the compound of formula (I) is
2-[(3,4-Dichlorophenyl)acetyl]-1,2,3,4-tetrahydro-1-(1-pyrrolidinyl)methyl]-5-isoquinolinol;
or a physiologically acceptable salt thereof.